Europäisches Patentamt

European Patent Office

Office européen des brevets

(11) Publication number: **0 192 321**
**A2**

## (12) EUROPEAN PATENT APPLICATION

(21) Application number: **86300179.8**

(22) Date of filing: **13.01.86**

(51) Int. Cl.⁴: **A 61 K 39/35**
**A 61 K 39/36, A 61 K 9/32**
**A 61 K 9/52**

(30) Priority: **19.01.85 GB 8501400**
**23.10.85 GB 8526133**

(43) Date of publication of application:
**27.08.86 Bulletin 86/35**

(84) Designated Contracting States:
**BE CH DE FR GB IT LI LU NL SE**

(71) Applicant: **BEECHAM GROUP PLC**
**Beecham House Great West Road**
**Brentford Middlesex TW8 9BD(GB)**

(72) Inventor: **Doughty, David George**
**2 Tonbridge Close Woodmansterne**
**Banstead Surrey SM7 3JD(GB)**

(72) Inventor: **Al-Janabi, Ismail**
**23 Miles Road**
**Epsom Surrey KT19 9AA(GB)**

(74) Representative: **Blake, John Henry Francis et al,**
**Beecham Pharmaceuticals Great Burgh Yew Tree**
**Bottom Road**
**Epsom, Surrey KT18 5XQ(GB)**

(54) **Enteric coated allergen pharmaceutical composition.**

(57) An enteric-coated allergen composition comprises a dispersion of allergen particles, typically freeze-dried allergen extracts, in a bulking agent which is a non-reducing sugar or a polyhydric alcohol; and a coating of an alkali or water soluble, acid insoluble polymer.

EP 0 192 321 A2

B1763

TITLE MODIFIED
see front page

## COMPOSITIONS

This invention relates to compositions containing allergens and their use in the therapy of allergic conditions in humans or animals.

Many people elicit characteristic respiratory symptoms to common aeroallergen materials such as pollens and house dust. Such allergic symptoms have been conventionally treated by the administration to the sufferer of repeated, gradually increasing doses of the relevant allergen to build up resistance to the allergen.

Oral administration of aqueous allergen vaccines has been employed for many decades, particularly for use with children. Although the mechanisms of action of such vaccines remain uncertain, there are several disadvantages in their use. Firstly, the allergenic proteins can be degraded rapidly by gastric secretions, thereby reducing the access of the proteins to lymphoid tissue in the lower gut. Secondly, possible adverse side effects can result from allergenic exposure to buccal, oesophageal and tracheal tissue.

It has been proposed that ragweed pollen (New Orleans Med. Surg. J. 1942, 94, 390-393) and grass pollen (Allergy 1983, 38, 561-564) may be administered orally in enteric-coated tablets to avoid gastric digestion of the allergens.

A novel enteric-coated allergen composition has now been developed which reduces or overcomes the above disadvantages.

According to the present invention there is provided a pharmaceutical composition comprising a dispersion of allergen particles in a bulking agent consisting of a non-reducing sugar or a polyhydric alcohol; and a coating of an alkali or water soluble, acid insoluble polymer.

The pharmaceutical composition may also comprise a pharmaceutically acceptable carrier.

The pharmaceutical composition of the invention has the advantage of being partially protected from the degradative effects of acid/pepsin environments for extended periods, while allowing rapid release of the allergen material at approximately neutral pH.

Examples of suitable bulking agents include $\alpha,\alpha$-trehalose, sucrose, sorbitol, xylitol and mannitol.

Preferably the bulking agent is mannitol.

Suitable polymers for use in the coating include hydroxy propyl methyl cellulose, hydroxy propyl methyl cellulose phthalate, cellulose acetate phthalate, polyvinyl acetate phthalate, or a methacrylate or acrylate polymer or copolymer. Preferred polymers are methacrylic acid/methyl methacrylate copolymers such as Eudragit L or Eudragit S (Eudragit is a trade mark of Rohm and Haas).

The weight ratio of allergen to bulking agent in the composition can vary within wide limits, but a preferred ratio is from 0.01 to 20%w/w (allergen: bulking agent).

Typically the allergen particles are allergen extracts obtained by extracting the allergen with a solvent. Usually an aqueous extracting solvent is used, which is then dialysed and freeze dried to remove impurities. Suitable allergens from which the extract can be obtained include pollens, such as grass pollens; weed pollens, such as ragweed; tree pollens, such as birch tree; house dust mites and venoms, such as bee venom, and other allergens of animal origin such as hair, fur and dander. Suitable extracting solvents include distilled water and ammonium bicarbonate solutions of various strengths, optionally containing phenol, phenol phosphate, buffer saline or diethyl ether. Preferably the extracting solvent is an ammonium bicarbonate solution.

The composition is suitably in the form of granules comprising a dispersion of allergen particles in the bulking agent. The granules may then be individually coated with the polymer coating material and optionally encapsulated in for example a gelatin capsule or compressed into a tablet or compact.

Alternatively the granules may be formed into a tablet or compact and the formed tablet coated with the polymer coating, or the granules may be encapsulated and the capsule coated with the polymer coating.

The granules are suitably of a size from 50 $\mu$m to 2mm.

The pharmaceutical compositions of the present invention are preferably in unit dose form for oral administration. Particularly suitable compositions are unit dosage tablets or capsules or powders presented in sachets. Suitable unit dosages contain from 8$\mu$g-20mg preferably from 200$\mu$g-5mg of allergen extract.

These dosages are suitable for administration to a human although the precise dosage will depend upon the severity of the condition and the size of the patient and will be determined by a physician. For example a 70 kg adult human may be administered approximately 5 mg of allergen.

In accordance with conventional pharmaceutical practice, other pharmaceutically acceptable agents such as diluents, fillers, disintegrants, wetting agents, lubricants, colourants, flavourants or the like can be incorporated into the composition of the invention. Examples of such agents include microcrystalline cellulose, starch, sodium starch glycollate, polyvinylpyrrolidone, polyvinyl-polypyrrolidone, magnesium stearate, sodium lauryl sulphate, and the like.

Normally, the allergen particles are the sole therapeutic substance present in the compositions of this invention.

Where the composition is in the form of a tablet or compact, it may further comprise a conventional compression aid such as lactose, dicalcium phosphate dihydrate, starch, microcrystalline cellulose or dextrates.

Preferably the compression aid comprises dextrates such as the spray-dried dextrates sold under the trade mark Emdex.

In addition the composition of the invention may further comprise a conventional buffering agent, such as an amino acid or inorganic salt of, for example glycine, alanine, phosphates, citrates, lactates or acetates. Suitable inorganic salts include the alkali metal salts (such as sodium) and the alkaline earth metal salts.

The pharmaceutical compositions of the invention may be prepared by film coating a dispersion of allergen particles in bulking agent, in the form of granules, or tablets, or compacts or capsules containing such granules, with a solution of alkali or water soluble, acid insoluble polymer in an inert solvent, and removing the solvent.

Suitable inert solvents for the polymer include methanol, ethanol, propanol, isopropanol, acetone, dichloromethane and water or mixtures thereof.

Preferably the solvent comprises water, a mixture of methanol and dichloromethane or a mixture of isopropanol and acetone.

Additional conventional agents such as plasticisers, anti-adherents and colourants may be included in the coating solution and thus form part of the final coating.

Examples of suitable plasticisers include phthalate esters such as diethylphthalate, polyethylene glycols, triacetin, castor oil, acetylated monoglycerides, acetyltriethylcitrate, and propylene glycol, of which triacetin and propylene glycol are preferred.

Examples of suitable anti-adherents include magnesium stearate, talc and fumed silica. A preferred anti-adherent is fumed silica.

- 6 -

Examples of suitable colourants include aluminium lakes of water soluble dyes or inorganic pigments such as iron oxide or titanium dioxide.

The granules of the allergen dispersed in bulking agent may be prepared by freeze drying allergen particles in the presence of the bulking agent. For example, a freeze dried allergen extract, preferably re-dissolved in distilled water, is mixed with a solution of the bulking agent, typically mannitol, preferably in distilled water, and the mixture is freeze-dried. The product is milled or screened to provide granules of the required size.

Tablets or compacts may be prepared by compression of these granules. Preferably the granules are combined with a compression aid and/or a buffering agent as well as any of the other conventional agents discussed above, prior to compression. Suitably the granules make up from 1-80% w/w of the final tablet. After compression, the tablets or compacts may be provided with the enteric coating.

Thus the present invention also provides a method of making an enteric coated allergen composition which comprises forming a composition by dispersing allergen particles in a bulking agent which is a non-reducing sugar or a polyhydric alcohol, and enveloping the composition with a coating of an alkali - or water-soluble, acid-insoluble polymer.

Further according to the present invention there is provided a method of treating allergic conditions in humans or animals by administering an effective non-toxic amount of a composition of the invention.

The following Examples illustrate the invention.

The allergen extracts used in the Examples are as follows.  All materials were dialysed following extraction and then freeze dried.

(a)  B2 Grass Pollen Extract (Bencard)

This was prepared from a mixture of equal weights of the following grass pollens:

| | |
|---|---|
| Bent | Agrostis tenuis |
| Brome | Bromus mollis |
| Cocksfoot | Dactylis glomerata |
| Crested Dogstail | Cynosurus cristatus |
| False Oat | Arrhenatherum elatius |
| Meadow Fescue | Festuca pratensis |
| Meadow Foxtail | Alopecurus pratensis |
| Meadow Grass | Poa pratensis |
| Rye Grass | Lolium perenne |
| Timothy | Phleum pratense |
| Sweet Vernal | Anthoxanthum odoratum |
| Yorkshire Fog | Holcus lanatus |

(b)  B2/Cultivated Rye Pollen Extract

This was made from a mixture of grass pollens containing 12 parts by weight of B2 grass pollens and 1 part weight of cultivated rye cereal pollen (Secale-cereale).

(c) B2/Bermuda Grass Pollen Extract

This was made from a mixture of grass pollens containing 9 parts by weight of B2 grass pollen and 1 part weight of Bermuda grass pollen (Cynodon dactylon).

(d) House Dust Mite Extract

This was made from a culture of Dermatophagoides pteronyssinus.

Example 1

A thirteen grass pollen (B2/Bermuda Grass) extract dissolved in distilled water was mixed with a solution of mannitol in distilled water. The mixture was freeze dried to provide a composition of extract/mannitol in the ratio 5:95 by weight. After screening, the resultant allergen/mannitol granules were blended with microcrystalline cellulose in the ratio 1:4 by weight in a mixer, and the mixture compressed on a suitable machine fitted with normal concave punches (5mm.) to yield compacts with a weight of 50mg and hardness 4KP, containing 500μg approximately of pollen extract.

Coating of the compacts was achieved by spraying a coating suspension onto a moving bed of tablets until the weight of a tablet had increased, on average by about 10mg. The coating suspension consisted of the following:

|  | g |
|---|---|
| Eudragit L100 | 120 |
| diethylphthalate | 16 |
| magnesium stearate | 24 |
| acetone/IPA (1:1 $^v/_v$) to | 2000 |

Example 2

A thirteen grass pollen extract (B2/Cultivated Rye pollen) was freeze dried together with mannitol in the ratio 5:95 by weight as in Example 1. After screening, the resultant allergen/mannitol granules were blended in a mixer with a mixed phosphate buffer (a mixture of mono and di-sodium phosphate which gives a buffer of pH 6), magnesium stearate and 'Emdex' (spray dried dextrates). The mixture was compressed in a tabletting machine fitted with normal concave punches of various diameters to produce round normal biconvex tablets. The components were mixed in the weight proportions shown in the following table to produce compacts containing 1 mg, 200 µg, 40 µg and 8 µg of allergen:

| Component | Parts by Weight | | | |
|---|---|---|---|---|
| allergen/mannitol | 20 | 4 | 0.8 | 0.16 |
| mixed phosphate buffer | 34 | 29 | 22 | 14 |
| magnesium stearate | 1.7 | 1.5 | 1.1 | 0.7 |
| 'Emdex' to | 170 | 145 | 110 | 70 |
| Tablet Size | 7.0mm | 6.5mm | 6.0mm | 5.0mm |
| Allergen content | 1 mg | 200µg | 40µg | 8µg |

- 11 -

0192321

The compacts were coated by spraying a coating suspension onto a moving bed of tablets until tablet weight had increased by about 15%. The coating suspension contained:

|  | % Solids by weight |
|---|---|
| methacrylic acid-methyl methacrylate copolymer ('Eudragit' L100) | 79 |
| Colloidal silica ('Aerosil' 200) | 12 |
| glyceryl triacetate (triacetin) | 9 |

suspended in a mixture of 28.4 parts methanol and 71.6 parts dichloromethane.

Example 3

The procedure of Example 2 was repeated to prepare coated tablets of similar compositions containing as allergen : House dust mite.

The enteric-coated allergen compositions of this invention are designed to protect the allergens from the degradative effects of the acid/pepsin medium of the stomach. In order to demonstrate the degree of protection offered by the enteric coat a test was devised based on the BP/USP distintegration test for enteric-coated tablets.

Tablets prepared in accordance with Examples 2 and 3 were placed individually into 20ml universal bottles containing either acid/pepsin (2g Nacl, 3.2g pepsin and 7ml concentrated HCl in 1 litre distilled water, pH 1.2) or distilled water. The bottles were roller-mixed at 37°C for 2 hours. The tablets were removed, rinsed with distilled water and dried with a tissue. They were then crushed and the contents solubilised with distilled water and 1% BSA (bovine serum albumin) by roller-mixing at room temperature for 15 minutes. Aliquots were then diluted with buffer to give the appropriate extract concentration for RAST assay.

Intact allergen remaining after the test was compared with that in tablets exposed to distilled water using RAST inhibition assay.

RAST - inhibition assay principle

Human IgE specific for allergens is neutralised by the allergens present in a known or unknown sample of allergens. Any of the remaining specific IgE is detected by contacting the mixture with a covalently bonded conjugate of cellulose paper discs and allergens and then contacting the new conjugate with an aqueous solution of $^{125}$I rabbit antihuman IgE. Potency is measured in units relative to a freeze dried reference preparation of allergen.

The results indicate that in the tested tablets D. Pteronyssinus (Table 1) and grass pollen (Table 2) are substantially protected against the degradative effects of the gastric conditions.

Table 1

Degree of protection against acid/pepsin of D. pteronyssinus enteric coated tablets (Example 3) (results expressed as Quality Assurance Units RAST per tablet).

RAST assay results

| gastric conditons | | distilled water | |
|---|---|---|---|
| individual tablets | combined estimate | individual tablets | combined estimate |
| 785.4 | | 1036.2 | |
| 825.6 | | 971.5 | |
| 1070.4 | | 962.1 | |
| 1236.9 | 1096.9 | 1177.4 | 1210.7 |
| 1311.2 | | 1684.9 | |
| 1351.6 | | 1431.9 | |

Table 2

Degree of protection against acid/pepsin of grass pollen extract enteric coated tablets (Example 2) (results expressed as Quality Assurance Units RAST per tablet).

| gastric conditions | | distilled water | |
|---|---|---|---|
| individual tablets | combined estimate | individual tablets | combined estimate |
| 37.8 | | 38.6 | |
| 33.2 | | 45.4 | |
| 42.8 | 38.9 | 67.5 | 49.6 |
| 36.1 | | 50.1 | |
| 43.2 | | 44.5 | |
| 40.0 | | 51.7 | |

- 1 -

CLAIMS                                                    C

1. A pharmaceutical composition comprising a dispersion of allergen particles in a bulking agent which is a non-reducing sugar or a polyhydric alcohol; and a coating of an alkali or water-soluble, acid-insoluble polymer.

2. A composition according to claim 1 in which the allergen particles are particles of a freeze-dried allergen extract.

3. A composition according to claim 1 or 2 in which the bulking agent is $\alpha,\alpha$-trehalose, sucrose, sorbitol, xylitol or mannitol.

4. A composition according to claim 3 in which the bulking agent is mannitol.

5. A composition according to any one of claims 1 to 4 in which the coating polymer is hydroxy-propyl methyl cellulose, hydroxy-propyl methyl cellulose phthalate, polyvinyl acetate phthalate, or a methacrylate or acrylate polymer or copolymer.

6. A composition according to claim 5 in which the coating polymer is a methacrylic acid-methyl methacrylate copolymer.

7. A composition according to any one of claims 1 to 6 in which the allergen is selected from grass pollens, tree pollens, weed pollens, house dust mites, venoms, animal fur, hair or dander.

8.    A composition according to any one of claims 1 to 7 further comprising a pharmaceutically acceptable carrier.

9.    A composition according to any one of claims 1 to 8 in which the allergen particles/bulking agent dispersion is in the form of granules and the granules are contained in a capsule or tablet coated with the coating agent.

10.    A composition according to any one of claims 1 to 8 in which the allergen particles/bulking agent dispersion is in the form of granules and the granules are coated with the coating  agent.

11.    A composition according to claim 10 in which the coated granules are held in a capsule or incorporated in a tablet.

12.    A method of making an enteric coated allergen composition which comprises forming a composition by dispersing allergen particles in a bulking agent which is a non-reducing sugar or a polyhydric alcohol, and enveloping the composition with a coating of an alkali- or water-soluble, acid-insoluble polymer.